(19) **Europäisches Patentamt European Patent Office Office européen des brevets**

(11) **EP 1 599 143 B1**

(12) **EUROPEAN PATENT SPECIFICATION**

(45) Date of publication and mention of the grant of the patent:
**29.01.2014 Bulletin 2014/05**

(21) Application number: **04707425.7**

(22) Date of filing: **02.02.2004**

(51) Int Cl.:
***A61B 17/34*** *(2006.01)*

(86) International application number:
**PCT/US2004/002971**

(87) International publication number:
**WO 2004/069035 (19.08.2004 Gazette 2004/34)**

(54) **SYSTEM FOR RAPID PLACEMENT OF CHEST TUBES**

SYSTEM FÜR DIE SCHNELLE PLATZIERUNG VON THORAXTUBEN

SYSTEME DE MISE EN PLACE RAPIDE DE TUBES DANS LA POITRINE

(84) Designated Contracting States:
**AT BE BG CH CY CZ DE DK EE ES FI FR GB GR HU IE IT LI LU MC NL PT RO SE SI SK TR**

(30) Priority: **31.01.2003 US 444345 P**

(43) Date of publication of application:
**30.11.2005 Bulletin 2005/48**

(73) Proprietors:
• **Simpson, Philip J.**
  **Escondido, CA 92025 (US)**
• **Matsuura, David G.**
  **Encinitas, CA 92024 (US)**
• **Gillespie, Walter Dean**
  **San Diego, CA 92109 (US)**
• **Salvino, Chris K.**
  **Chicago, IL 60611 (US)**

(72) Inventors:
• **SIMPSON, Phlip, J.**
  **Escondido, CA 92025 (US)**

• **MATSUURA, David, G.**
  **Encinitas, CA 92024 (US)**
• **GILLESPIE, Walter, Dean**
  **San Diego, CA 92109 (US)**
• **SALVINO, Chris, K.**
  **Chicago, IL 60611 (US)**
• **TRINCHERA, Jim**
  **Leucadia, CA 92024 (US)**

(74) Representative: **Freeman, Avi et al**
  **Beck Greener**
  **Fulwood House**
  **12 Fulwood Place**
  **London WC1V 6HR (GB)**

(56) References cited:
  WO-A1-03/008020    US-A- 5 098 392
  US-A- 5 221 263    US-A- 5 429 598

## Description

### Field of the Invention

The present invention relates to apparatus for performing a rapid tube thoracostomy, and more particularly relates to apparatus for performing a rapid tube thoracostomy using conformable tubes and cannula.

### Background of the Invention

A trocar such as known e.g. from WO-A-03/008020 generally comprises an obturator and a cannula. The obturator has a pyramid-shaped piercing tip at one end, and moves the piercing tip into tissue to form a hole to provide access to a body cavity or a target tissue. The cannula is located around the obturator. The cannula is inserted into the body cavity together with the obturator through the hole formed by the piercing tip. Such a trocar, therefore, forms a pathway in the inside of the cannula for inserting an endoscope or a surgical tool into the body cavity, by extracting or withdrawing the obturator from the cannula, which is inserted into the body cavity. Known methods of sealing the tissue to the cannula include the use of sutures and/or adhesive tape in order to maintain the position of the cannula and provide a fluid and air tight seal. However, this method fails to provide adequate barrier or an appropriate seal for fluid and/or gases. Therefore what is needed is a system and method for providing an air and fluid tight seal without the use of sutures and/or adhesive tape.

### Summary of the Invention

The present invention provides a system for safely and easily performing a rapid tube thoracostomy. Tube thoracostomy is a method for allowing the sterile drainage of fluid or air from the pleural space utilizing a semi-rigid drainage tube. In at least some implementations, the present invention also minimizes the need for exposed sharp instruments such as scalpels.

According to an embodiment of the present invention, there is provided a rapid tube insertion system for inserting tubes into a pleural space in a chest, the system comprising: a cutting device having an ovate probe tip which extends distally, a compliant cannula capable of being placed over the probe tip, the end of the probe tip extending beyond the cannula, the cannula having an ovate shape and being capable of being inserted into an incision into the pleural space, a chest tube having a distal end adapted to be inserted through the compliant cannula and a proximal portion extending outwardly therefrom, and the cannula being capable of being passed over the proximal portion of the tube.

In embodiments, the tube is a drain tube. In embodiments, the compliant cannula has a portion thereof formed of a less compliant material than the remainder thereof. In embodiments, the tube includes longitudinal reinforcement to prevent kinking. The probe tip of the cutting device has an ovate cross-section. The compliant cannula has a preformed ovate cross-section.

### Brief Description of the Figures

Figure 1 illustrates in perspective view generally an insertion gun, a cannula and chest tube in accordance with one aspect of the present invention.

Figure 2 illustrates, in side elevation view, in addition to the system of Figure 1, a seal in accordance with one aspect of the present disclosure.

Figure 3 illustrates the gun of Figure 1 applied to an area of tissue characteristic of a patient needing aid, with the cannula on the probe of the gun.

Figure 4 illustrates the gun of Figure 1 inserted partly into the patient after appropriate cuts have been made.

Figure 5 illustrates the gun removed from the patient with the cannula remaining inserted into the patient.

Figure 6 illustrates the insertion of the chest tube through the cannula of Figure 5.

Figure 7 illustrates the chest tube inserted into the patient through the cannula.

Figure 8 illustrates the removal of the compliant cannula over the chest tube.

Figure 9 illustrates the application of a sealing element over the chest tube.

Figure 10 illustrates the sealing of the wound with the seal over the chest tube, thus completing the chest tube installation.

Figures 11A-11B show in cut-away view one implementation of the gun of Figure 1, with Figure 11B providing a detail view of the cutting tip of the gun.

Figure 12 shows in cut-away view certain details of the implementation of Figure 11A.

Figure 13 illustrates in cut-away view the gun of Figure 11A with the trigger at approximately mid-point.
Figure 14 illustrates in more detail an implementation of the gun of Figure 13.
Figure 15A illustrates the implementation of the gun of Figure 11A with the trigger fully retracted.
Figure 15B illustrates in detail cut-away view the cutting tip of Figure 15A.
Figures 16A-16D illustrate the eccentric nature of the cutting tip of the gun.
Figures 17A-17D illustrate cross-sectional view of various chest tubes, both in compressed and uncompressed views.
Figures 18A-18E illustrate various details of some examples of chest tubes in accordance with the present invention.
Figures 19A-19B illustrate perspective and cross-sectional side views of a seal in accordance with the disclosure.
Figure 20 illustrates an adhesive method for attachment of the chest tube assembly to a patient.
Figures 21 A-21C illustrate, respectively, a side elevation view of a snap lock fitting for attachment of the chest tube to a patient, a cross-sectional side view, and a perspective view of the snap lock fitting itself.
Figures 22A-22B illustrate in cross-sectional side view the attachment of the snap lock fitting of Figures 21A-21C to a patient.

## Detailed Description of the Invention

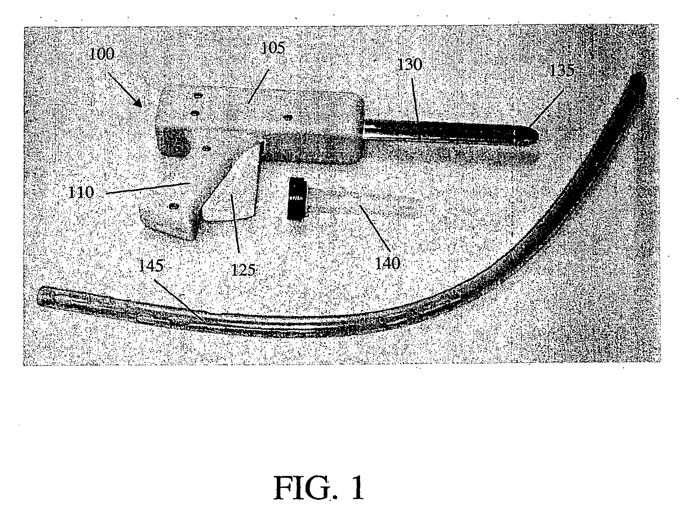

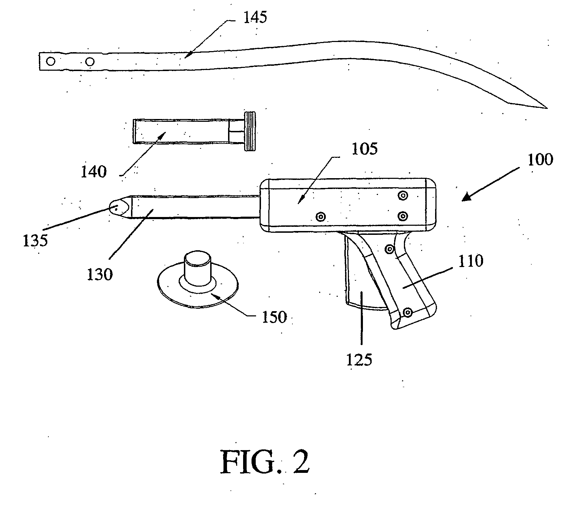

Referring first to Figures 1 and 2, a system for chest tube insertion in accordance with the present invention is shown generally. A chest tube insertion device 100, which may also be thought of as a cutting and insertion gun, includes a housing 105, a handle 110 with a trigger 125, a probe tip 130 having a cutting tip 135 at the distal end thereof, a cannula 140, a chest tube 145 and a seal 150.

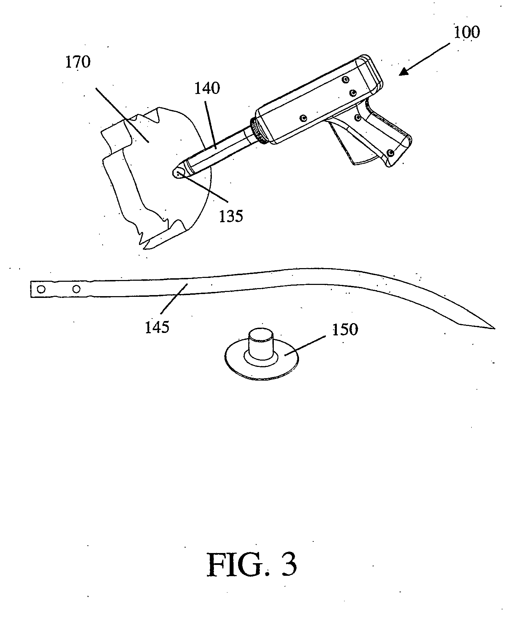

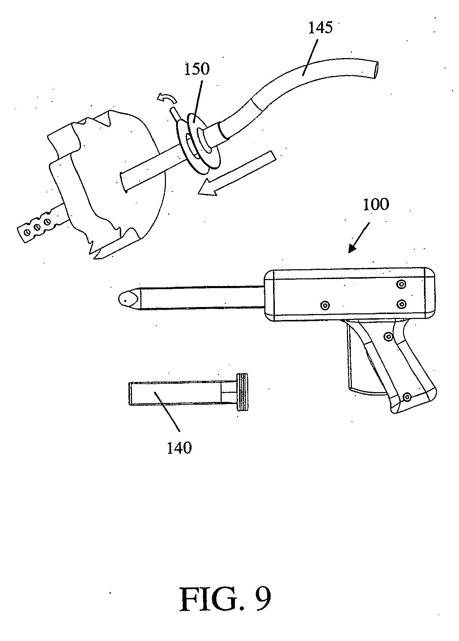

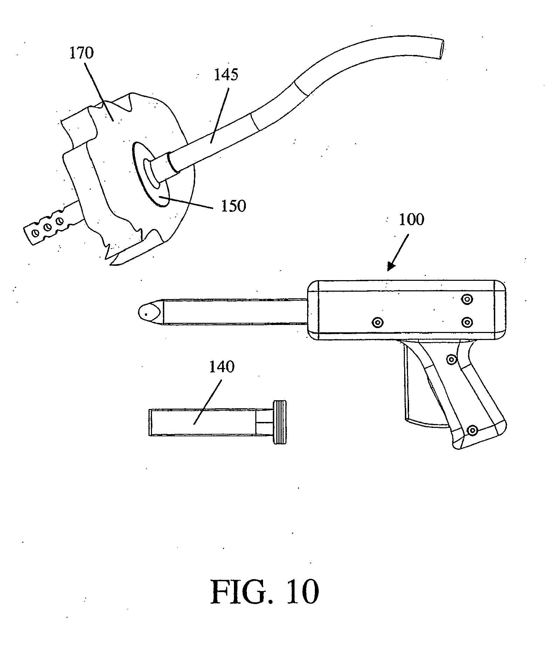

Referring next to Figures 3-10, the process for inserting a chest tube according to the present disclosure can be better appreciated. The chest tube insertion procedure using this system is safer, faster, and easier than other known methods. Although not a required part of the present invention, clinicians considering insertion of a chest tube typically include the steps of selecting a site, preparing the patient and then draping the area, followed by anesthetizing the site. The anesthesia can be of any acceptable type; one typical approach is 5 to 15 ml of 1% lidocaine delivered through a syringe and small gauge needle.

Following the foregoing preliminary steps, the chest tube insertion procedure in accordance with the present disclosure proceeds as follows:

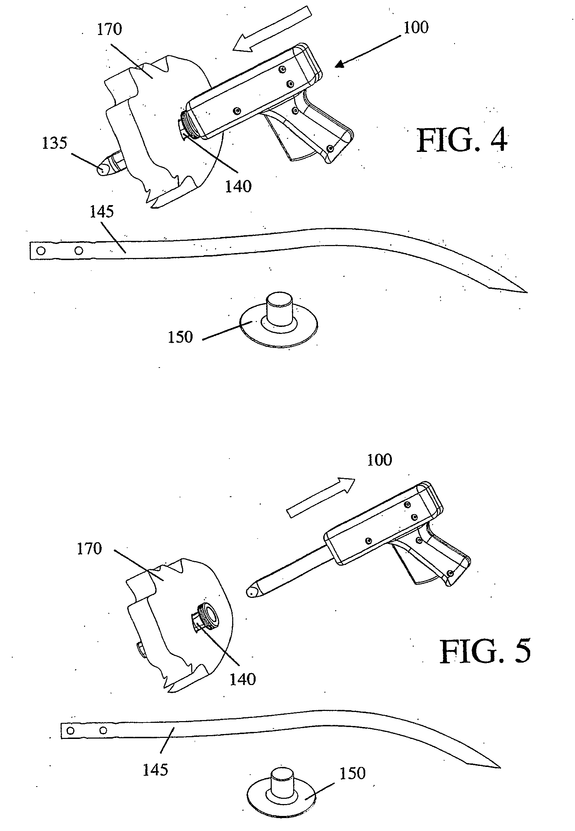

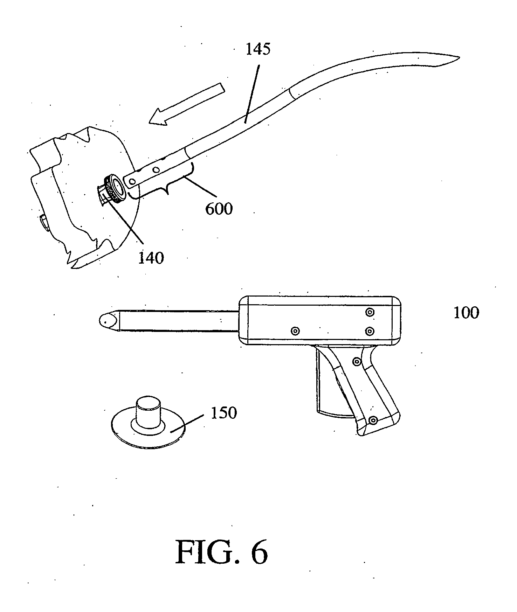

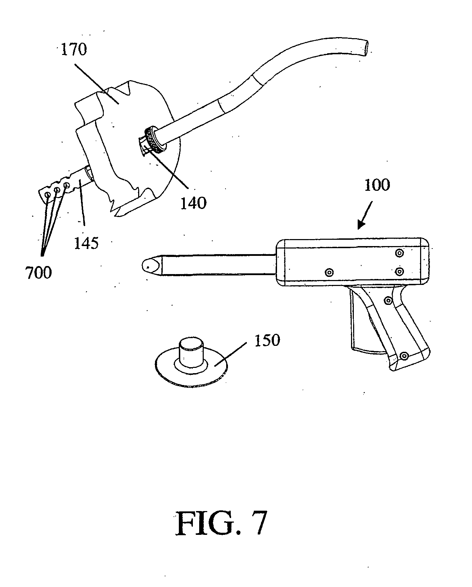

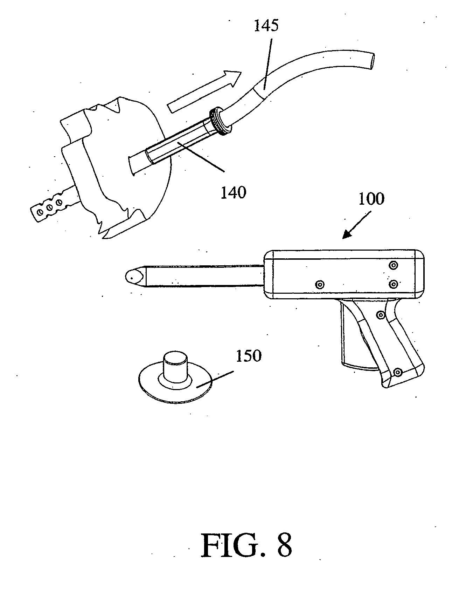

1. Make an incision through skin to the pleural space with the chest tube insertion device, or gun, 100. Start by placing a cannula 140 over the probe tip or shaft 130 of the device 100 until the cutting tip 135 extends beyond the cannula 140.
2. Place the device 100 against the patient's target tissue 170 as shown in Figure 3. Visually aligning the shaft 130 of the device in the desired direction. Firmly press the distal tip 135 into the skin and actuate the trigger 125. Actuation of the trigger will initiate a cutting event, creating an incision 175, as shown in Figure 4. Each cutting event will cut approximately 1 mm in depth as long as the cutting tip is maintained appropriately against the tissue 170. As the device 100 cuts through skin, the device 100 may be used as a blunt dissection device, and may be thought of as a blunt tip obturator.
3. Once the tip of the probe 130 extends into the pleural cavity in the desired amount, the cannula will also extend into the cavity as shown in Figure 4. Withdrawal of the gun 100 from the incision 175 can be achieved while leaving the cannula 140 in place within the patient as shown in Figure 5.
4. As shown best in Figure 6, introduce the distal tip 600 of the chest tube 145 into the cannula 140.
5. Advance the tube 145 until all of the transverse drain holes 700 of the chest tube 145 are within the pleural space, as shown in Figure 7.
6. Withdraw the cannula 140 over the chest tube 145 while holding the chest tube 145 in place, best shown in Figure 8.

Then, as is typical of thoracostomies, the clinician will typically take the additional steps of suturing the skin on both sides of the chest tube and tying the tube in place with the tag ends of the suture; applying sterile petroleum gel over the incision to create an airtight seal and cutting notches in sterile gauze to fit around the chest tube, followed by securing the gauze and tube in place using a suitable surgical tape.

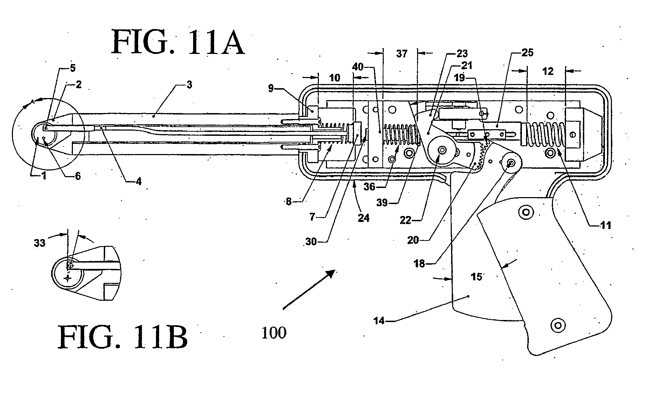

From the foregoing, a method of rapidly placing a chest tube according to the disclosure can be appreciated. However, the chest insertion device 100 may be implemented in any of a variety of designs, just as the cutting tip 135 may be implemented in a variety of ways. Several of these implementations are described in connection with Figures 11A through 15.

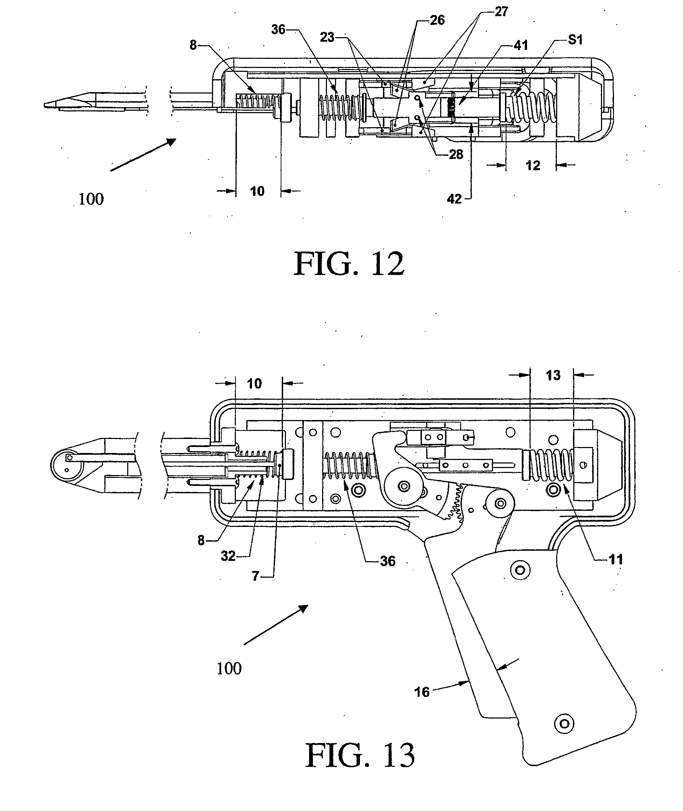

Referring next to Figures 11A-11B and 12, a first implementation of the chest tube insertion device shown generally at 100 may be better appreciated. An eccentrically mounted circular blade 1 is housed inside a bearing block 2 and is attached to the bearing block 2 by an axle 6. The blade 1 need not be an eccentrically mounted circular blade. The bearing block 2 is located at the distal end of a shaft or probe 3, which is similar to the probe 130 of Figure 1. The blade 1 is also connected to the distal end of an input rod 4 by a pivot pin 5. A compression spring 8, is constrained at a compressed height 10, by a flange 7 at the proximal end of the input rod 4 and a wall 9 inside the mechanism housing. The force from the compression spring 8, translates through the input rod 4 to the eccentrically mounted blade 1 through the pivot pin 5. The force acting on the pivot pin 5 results in a moment about the axle 6 which keeps the blade 1 recessed inside the bearing block 2 until the operator initiates a cutting event.

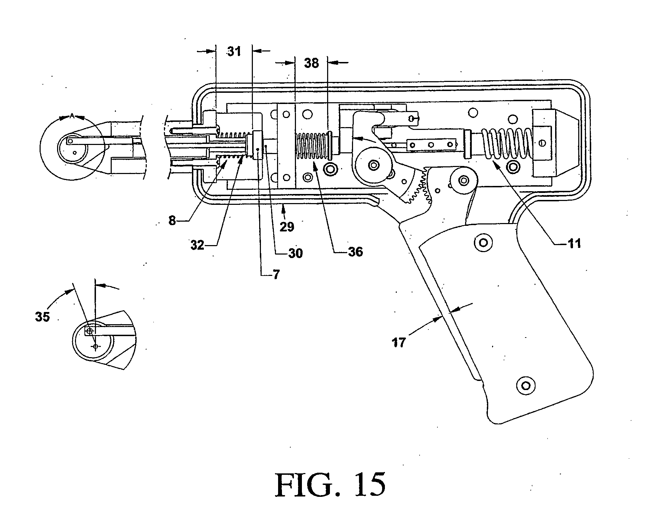

To initiate a cutting event, the operator moves an input lever or trigger 14, which is similar to the trigger 125 of Figure 1, from a stationary position 15, as shown in Figures 11A-11B and the more detailed view of Figure 12, through an intermediate position 16 as shown in Figure 13 to a final position 17 shown in figure 15. As the input lever 14 is moved; it pivots about a lever axle 18. The angular rotation of the lever 14 is translated through one segment of circular gear teeth 19 mounted concentric to the lever axle 18, to a meshing segment of circular gear teeth 20 attached to a cam 21. The cam 21 is allowed to rotate about a shaft 22 as it is motivated to do so by motion of the gear teeth 20.

The cam 21 profile is exaggerated through a pair of elongated members 23 which contact a momentum storage mass 25 through a matching pair of latches 26 which are attached to the mass 25 by pins 28. The latches 26 are able to rotate about the pins 28 that connect them to the mass 25. The mass 25 is constrained to move only longitudinally on an axis co-linear with the shaft 3. Angular cam motion is translated to distal-to-proximal linear motion of the mass 25 as the cam 21 rotates from a stationary position 24 shown in Figure 11 A to a final position 29 as shown in Figure 15. In the particular implementation shown, the mass 25 is moved rearward or toward the proximal end of the device 100.

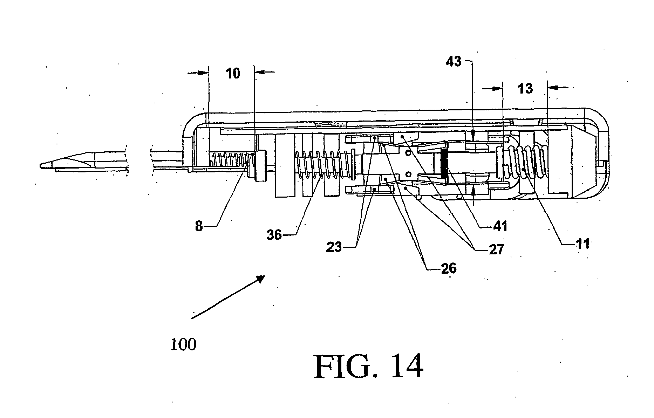

Distal-to-proximal linear motion of the mass 25 causes the angled outer surfaces of the matching pair of latches 26 to encounter stationary protrusions 27. As detailed in figure 14, the protrusions 27 act on the angled outer surfaces of the latches 26 so that the latches 26 rotate about their pivot pins 28 until they no longer contact the cam members 23. Simultaneously, the mass 25 compresses a spring 11 from its free length 12 as depicted in Figure 11, as it travels proximally, to a compressed length 13 as depicted in Figure 13. Release of the latches 26 from the cam members, enable the resultant force created by compression of the spring 11 to act on the momentum storage mass 25 thereby accelerating it in a proximal to distal direction. Potential energy stored in the spring 11 at the compressed length 13 is converted to kinetic energy as the mass 25 is accelerated.

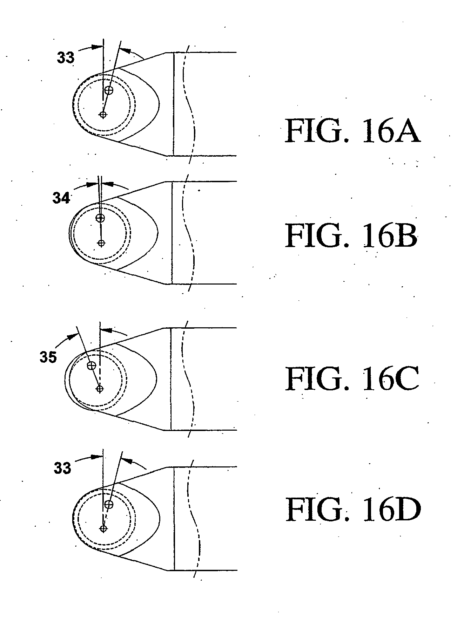

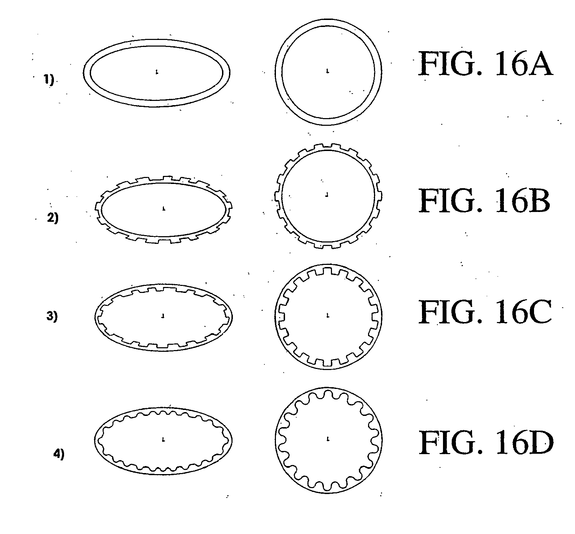

The proximal to distal motion of the mass 25 causes its distal most face 30 to strike the proximal end of the rod 4. The mass 25 and the rod 4 continue with a proximal to distal motion until the flange 7 strikes a travel limiting structure 32. The proximal to distal motion of the rod 4 acts on the blade 1 such that it rotates about the axle 6, which attaches the blade 1 to the bearing block 2. As the mass 25 and the rod 4 travel proximal to distally, the blade 1 rotates about the axle 6, which connects the blade 1 to the bearing block 2. Depicted in Figures 16A-D, as the blade 1 rotates, it emerges from the bearing block 2 from a stationary position to a fully exposed position when the rod 4 encounters the travel limiting structure 32.

As the momentum storage mass 25 travels in a proximal to distal direction it compresses a spring 36 constrained between a flange 39 and stationary internal structure 40. Force stored in the spring 36 created by compressing the spring 36 to a pre-loaded height 38 acts on the mass 25 once its proximal to distal motion has been halted by the travel limiting structure 32. The force generated by the spring 36 causes the mass 25 to move in a distal to proximal direction until equilibrium is achieved. With the mass 25 reset, the compression spring 8 that is in contact with the proximal end of the rod 4 acts on the rod 4 to move the rod 4 in a distal to proximal direction thereby recessing the blade 1 to a safe position inside the bearing block 2.

The operator resets the mechanism after initiating a cutting event by releasing the input lever 14. The input lever 14 then returns to the stationary position 15 by means of a spring (not shown) in contact with the input lever 14, causing the input lever 14 to rotate about the lever axle 18. Motion of the lever 14 causes the cam 21 to move to its stationary position 24. As the elongated members 23 move to their stationary positions the latches 26 attached to the mass 25 are acted on by an extension spring 41 to return the latches 26 to their position 42. The mechanism is now reset and ready for another operator initiated cutting event.

Optionally, an additional user control may be incorporated into the device 100 to hold the cutting element fully extended when actuated. This alternative control allows the clinician to optionally use the device as a sharp trocar as well.

One aspect of the chest tube insertion device is that the shaft 130 is, substantially ovate in cross section. This allows for passage of a larger bore transversely compliant cannula to be inserted between the ribs without dilating the rib cage. At room temperature, standard chest tubes are fairly diametrically compliant, across the transverse axis of the tube, and become much more compliant at body temperature. Therefore, standard chest tubes can be passed through a substantially ovate cannula. Thus, a larger chest tube can be inserted with significantly less pain to the patient. Optionally, the cannula may be pre-formed with an ovate cross section of a less compliant material.

Another aspect of the device is the use of preferentially compliant chest tubes. Standard chest tubes are formed with uniform wall thickness. These tubes are formed with walls heavy enough to prevent kinking across the transverse axis of the tube due to longitudinal bending loads. When using a substantially ovate cannula, the load required to pass the circular chest tube through the cannula, can be substantially reduced through the use of a tube that is preferentially more diametrically compliant, across the transverse axis, than standard tubes.

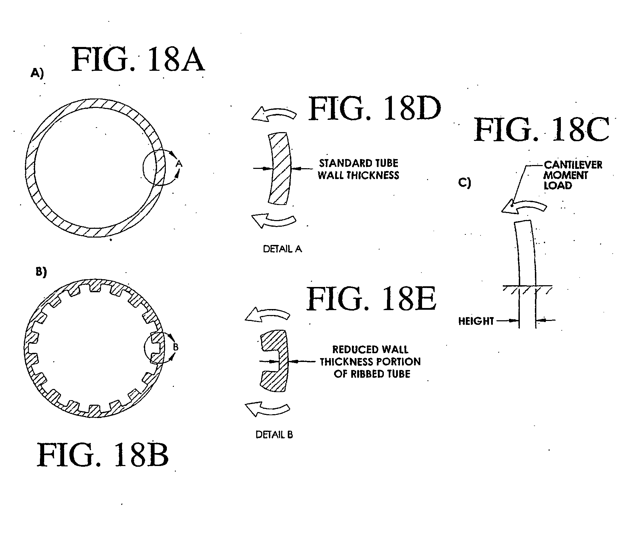

In some embodiments of the invention, it is desirable to use a generally thinner walled tube, which may for example be formed by extrusion, with walls formed with a multiplicity of longitudinal ribs, scallops or splines as shown in Figures 17A-17D. The ribs may be on the inside or outside of the tube, and may take any of a wide variety of shapes. The space between the ribs form flexure zones, thus allowing the tube to be more diametrically compliant than a standard tube. Another purpose of the ribs is to provide adequate section modulus to prevent transverse kinking of the tube when bent under normal loading conditions, such as shown by the equations set forth below and shown in Figures 18A-18E. Furthermore, design of interior ribs could provide patent lumens, to prevent complete shut off, even when exposed to extreme loading conditions. Additionally the ribs add sufficient section to provide adequate resistance to tensile loads.

**Cantilever-moment load**

$$\text{Modulus}_{\text{section}} := \frac{\text{Base} \cdot \text{Height}^3}{12}$$

$$\text{Moment} := 2 \cdot \frac{\text{deflection} \cdot \text{Modulus}_{\text{Elastic}} \cdot \text{Modulus}_{\text{section}}}{\text{Length}^2}$$

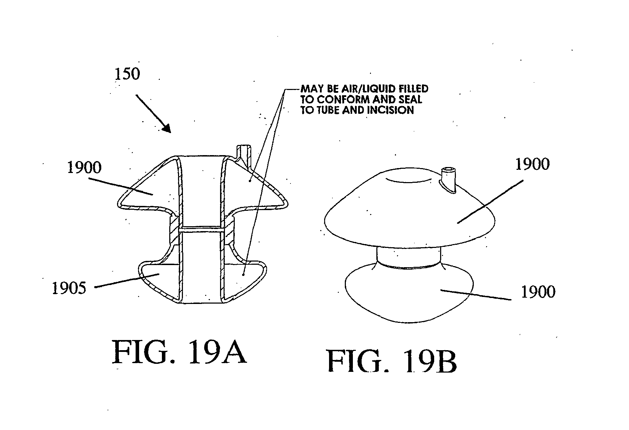

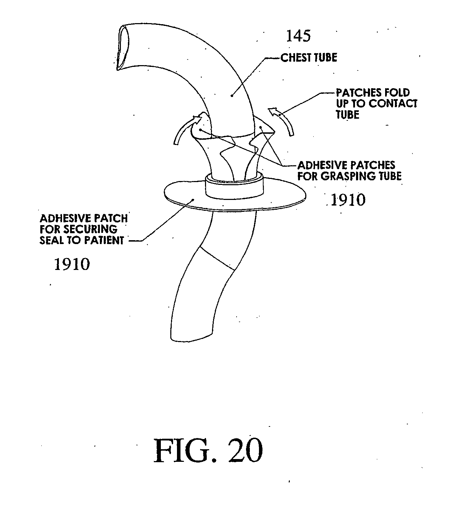

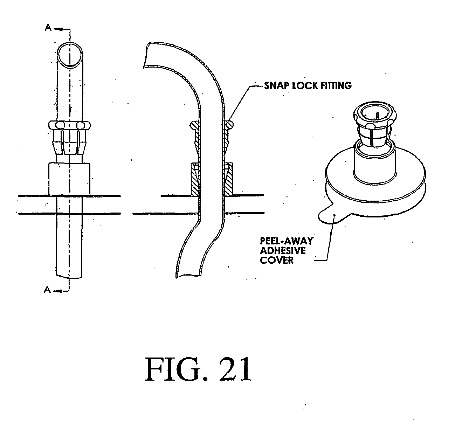

Another innovative aspect of the some embodiments of the system of the invention is the use of an elastomeric pneumo-seal, such as shown at 150 in Figure 2 and better seen in Figures 19A-19B. The term pneumo-seal is used herein to refer to a seal that acts as a barrier to prevent the flow of fluids and gases. The pneumo-seal 150 can be formed of a compliant material with upper flange 1900 and lower flange 1905, and may have pressure sensitive adhesive attachment areas 1910 as shown in Figures 20 and 21 to adhere to and seal to the patient and the chest tube. The pressure sensitive seal or barrier formed by the pneumo-seal is accomplished without stitches or sutures. The pneumo-seal may be pre-loaded and reside on the cannula 140 or may be used as a separate device.

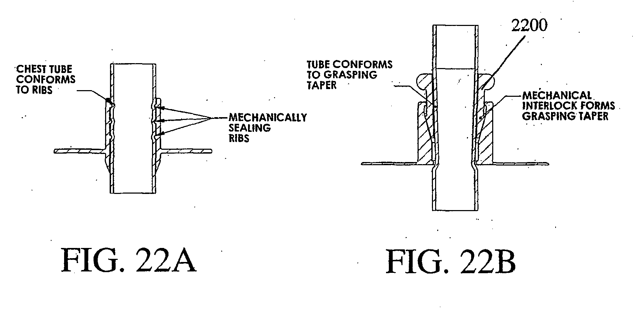

Alternatively, the pneumo-seal device 150 may have a fitting 2200 that mates to a matching fitting integrally formed with the chest tube or, as shown in Figure 22B, provided as a snap-lock fitting added to the chest tube. The purpose of the fitting is to mechanically attach the tube to the patient patch and to form an air-tight seal.

Alternatively, mechanical sealing ribs (Figure 22A) may also replace the adhesive contacting tube. The basic configuration of the pneumo-seal 150 can also be used for other cannulations into the body such as central venous lines and other drainage tubes. Optionally, the device may be molded from a transparent material, such as Pebax, to allow visualization of the wound through the device. The advantages of the pneumo-seal device include that it is faster than cutting bandages, requires no use of scissors, is faster than suturing or tying, requires no needles, does not require petroleum gel to form seal (thus allowing use of standard wound dressing tapes.

It will thus be appreciated that a new and novel method of chest tube insertion has been disclosed, as well as a new and novel chest tube insertion system and components thereof. Among the advantages offered by the use of one or more of implementations of the invention are a controlled depth of cut, a retractable blade offering increasing user and patient safety, greater safety for the clinician. Having fully disclosed a variety of implementations of the present invention, it will be appreciated by those skilled in the art that numerous alternatives and equivalents exist which do not materially alter the invention described herein. Therefore, the invention is not intended to be limited by the foregoing description, but instead only by the appended claims.

In an embodiment, the method further includes the step of using the probe tip as a blunt tip obturator. The step of providing a compliant cannula may include providing a cannula which is substantially ovate in shape. The step of providing a compliant cannula may include providing a cannula preformed to have an ovate cross-section. The compliant cannula may be formed of a relatively less compliant material. The step of providing a compliant cannula may include providing a tube preformed to have an ovate cross-section. The compliant cannula may be formed of a relatively less compliant material. The step of providing a tubeless compliant cannula may include providing a cannula preformed to have an ovate cross-section. The tubeless compliant cannula may be formed of a relatively less compliant material. The step of providing a tubeless compliant cannula may include providing a tube preformed to have an ovate cross-section. The compliant cannula may be formed of a relatively less compliant material. The step of providing a tube may include providing a tube which is preferentially compliant. The step of providing a tube may further include providing a tube which has adequate section modulus to prevent kinking. The incising step may includes incising the chest cavity. The step of providing a tube may include providing a chest tube. The incising step may include incising the abdominal cavity. The step of providing a tube may include providing an abdominal tube. The method may further include the steps of providing a seal and attaching the seal to the chest tube to form an air tight seal substantially where the seal contacts the body cavity. The step of providing a seal may include providing a pneumo seal which mates to the chest tube. The body cavity may be a vein. The tube may be a drainage tube. The tube may be inserted into a body cavity of a human being.

## Claims

1. A rapid tube insertion system for inserting tubes into a pleural space in a chest, the system comprising:

   a cutting device (100) having a probe tip (130) with an ovate cross-section, which probe tip extends distally,
   a compliant cannula (140) capable of being placed over the probe tip, the end of the probe tip extending beyond the cannula, the cannula having an ovate cross-section and being capable of being inserted into an incision into the pleural space,
   a chest tube (145) having a distal end adapted to be inserted through the compliant cannula and a proximal portion extending outwardly therefrom, and the cannula being capable of being passed over the proximal portion of the tube.

2. A system according to claim 1, wherein the tube is a drain tube.

3. A system according to claim 1 or 2, wherein the compliant cannula has a portion thereof formed of a less compliant material than the remainder thereof.

4. A system according to any one of the preceding claims, wherein the tube includes longitudinal reinforcement to prevent kinking.

## Patentansprüche

1. Schnelles Schlauch-Einführsystem zum Einführen von Schläuchen in einen Pleuralraum in einem Brustkorb, wobei das System Folgendes aufweist:

   eine Schneidvorrichtung (100), die eine Sondenspitze (130) mit einem eiförmigen Querschnitt aufweist, wobei sich die Sondenspitze distal davon erstreckt,
   eine nachgiebige Kanüle (140), die zum Anbringen über der Sondenspitze geeignet ist, wobei das Ende der Sondenspitze sich über die Kanüle hinaus erstreckt, die Kanüle einen eiförmigen Querschnitt aufweist und geeignet ist, durch einen Einschnitt in den Pleuralraum eingeführt zu werden,
   einen Thoraxdrainageschlauch (145) mit einem distalen Ende, das eingerichtet ist, durch die nachgiebige Kanüle eingeführt zu werden, und einem proximalen Abschnitt, der sich nach außen davon erstreckt und die Kanüle geeignet ist, über den proximalen Abschnitt des Schlauches geführt zu werden.

2. System nach Anspruch 1, wobei der Schlauch ein Drainageschlauch ist.

3. System nach Anspruch 1 oder 2, wobei die nachgiebige Kanüle einen Abschnitt aufweist, der aus einem weniger nachgiebigen Material gebildet ist als der restliche Abschnitt davon.

4. System nach einem der vorhergehenden Ansprüche, wobei der Schlauch eine Längsverstärkung zur Verhinderung des Abknickens aufweist.

## Revendications

1. Système d'insertion rapide du tube pour insérer des tubes dans un espace pleural d'une poitrine, le système comprenant :

un dispositif de coupe (100) ayant une pointe de sonde (130) avec une section transversale ovale, dont la pointe de sonde s'étend de manière distale,

une canule souple (140) apte à être placé sur la pointe de sonde, l'extrémité de la pointe de sonde s'étendant au-delà de la canule, la canule ayant une section transversale H ovale et étant susceptible d'être insérée dans une incision dans l'espace pleural,

un tube de poitrine (145) ayant une extrémité distale adaptée pour être insérée à travers la canule souple et une partie proximale s'étendant vers l'extérieur, et la canule étant susceptible d'être passée au-dessus de la partie proximale du tube.

2. Système selon la revendication 1, dans lequel le tube est un tube d'égouttement.

3. Système selon la revendication 1 ou 2, dans lequel la canule souple a une portion formée d'un matériau moins souple que le reste de la canule.

4. Système selon l'une quelconque des revendications précédentes, dans lequel le tube inclut un renforcement longitudinal pour empêcher la formation de noeud.

FIG. 1

FIG. 2

FIG. 3

170

100

135

140

145

150

FIG. 4

100

170

140

145

150

FIG. 5

FIG. 6

FIG. 7

FIG. 8

FIG. 9

# FIG. 10

# FIG. 11A

# FIG. 11B

100

# FIG. 12

# FIG. 13

FIG. 14

FIG. 15

FIG. 16A

FIG. 16B

FIG. 16C

FIG. 16D

EP 1 599 143 B1

1)
2)
3)
4)

FIG. 16A

FIG. 16B

FIG. 16C

FIG. 16D

22

A)  # FIG. 18A

# FIG. 18D

STANDARD TUBE
WALL THICKNESS

DETAIL A

# FIG. 18C

C)

CANTILEVER
MOMENT
LOAD

HEIGHT

B)

# FIG. 18E

REDUCED WALL
THICKNESS PORTION
OF RIBBED TUBE

DETAIL B

# FIG. 18B

150

1900

1905

MAY BE AIR/LIQUID FILLED
TO CONFORM AND SEAL
TO TUBE AND INCISION

1900

1900

FIG. 19A          FIG. 19B

145
CHEST TUBE

PATCHES FOLD
UP TO CONTACT
TUBE

ADHESIVE PATCHES
FOR GRASPING TUBE

1910

ADHESIVE PATCH
FOR SECURING
SEAL TO PATIENT

1910

FIG. 20

SNAP LOCK FITTING

PEEL-AWAY
ADHESIVE
COVER

# FIG. 21

CHEST TUBE
CONFORMS
TO RIBS

MECHANICALLY
SEALING
RIBS

FIG. 22A

TUBE CONFORMS
TO GRASPING
TAPER

2200

MECHANICAL
INTERLOCK FORMS
GRASPING TAPER

FIG. 22B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03008020 A